# EUROPÄISCHE PATENTSCHRIFT

(11) **EP 1 252 338 B1**
(45) Veröffentlichungstag und Bekanntmachung des Hinweises auf die Patenterteilung: **17.10.2007**
(21) Anmeldenummer: 01948905.3
(22) Anmeldetag: 18.01.2001
(51) Int. Cl.: C12Q 1/68

(54) **NUKLEINSÄURE-ISOLIERUNG AUS STUHLPROBEN UND ANDEREN BIOLOGISCHEN MATERIALIEN, DIE REICH AN INHIBITOREN SIND**
NUCLEIC ACID ISOLATION FROM STOOL SAMPLES AND OTHER INHIBITOR-RICH BIOLOGICAL MATERIALS
ISOLATION D'ACIDES NUCLEIQUES A PARTIR D'ECHANTILLONS DE SELLES ET D'AUTRES MATERIAUX BIOLOGIQUES RICHES EN INHIBITEURS

(30) Priorität: 04.02.2000 DE 10004927; 17.03.2000 DE 10013225
(43) Veröffentlichungstag der Anmeldung: 30.10.2002
(73) Patentinhaber: QIAGEN GmbH, 40724 Hilden (DE)
(72) Erfinder: SPRENGER-HAUSSELS, Markus, 42697 Solingen (DE)
(74) Vertreter: Weiss, Wolfgang
(86) Internationale Anmeldenummer: PCT/EP2001/000515
(87) Internationale Veröffentlichungsnummer: WO 2001/057247

(56) Entgegenhaltungen:
- EP-A- 0 811 694
- EP-A- 0 939 118
- EP-A- 1 044 984
- WO-A-00/42177
- WO-A-97/07239
- US-A- 5 817 798
- US-A- 5 910 407
- GOUVEA V. ET AL: "Identification of hepatitis E virus in clinical specimens: amplification of hydroxyapatite-purified virus RNA and restriction endonuclease analysis" JOURNAL OF VIROLOGICAL METHODS, Bd. 69, 1997, Seiten 53-61, XP000901005

## Beschreibung

Die Erfindung betrifft ein Verfahren zur Stabilisierung, Reinigung oder/und Isolierung von Nukleinsäuren aus Materialproben, insbesondere Stuhlproben, die Verunreinigungen und Inhibitoren bzw. Störsubstanzen enthalten können. Weiterhin wird ein für die Durchführung des Verfahrens geeigneter Reagenzienkit beschrieben.

Zahlreiche Beispiele aus verschiedenen Forschungsbereichen belegen die Bedeutung der Analyse von Nukleinsäuren aus biologischen Materialien, die mit Substanzen verunreinigt sind, welche Nukleinsäuren während der Lagerung schädigen und eine enzymatische Manipulation der Nukleinsäuren, z.B. Verdau mit Restriktionsenzymen oder Amplifikation durch Polymerase-Kettenreaktion (PCR) hemmen. Daher ist es für die Brauchbarkeit der in den biologischen Materialien enthaltenen Nukleinsäuren für weitere Analysen wichtig, daß diese Substanzen nur in sehr niedrigen Konzentrationen vorhanden sind oder gänzlich aus der Probe entfernt werden.

Eine besondere Bedeutung besitzt die Analyse von Nukleinsäuren aus fäkalen Proben. Eine wichtige medizinische Anwendung ist der Nachweis tumorspezifischer Veränderungen nukleärer Human-DNA aus Stuhl, die als Parameter bei der Frühdiagnose von Tumoren des Verdauungstraktes dienen können. Ebenso gewinnt der Nachweis bakterieller und viraler Infektionserreger aus Stuhlproben durch auf Nukleinsäuren basierende Testverfahren zunehmend an Bedeutung.

Für die Aufreinigung von Nukleinsäuren aus Stuhlproben ist die Anwendung einer Kombination verschiedener Reinigungsschritte wie Protease-Behandlung, Phenol/Chloroform-Extraktion, Bindung von Nukleinsäuren an Silika in Anwesenheit chaotroper Salze, Gelfiltration, Anionenaustauschchromatographie sowie Einsatz kationischer Detergentien bekannt. Die mit diesen Verfahren aus Stuhlproben isolierten Nukleinsäuren sind jedoch im allgemeinen instabil und verhalten sich oftmals problematisch in nachfolgenden enzymatischen Reaktionen wie z.B. PCR.

Ursache hierfür sind Substanzen, die zusammen mit der Nukleinsäure isoliert werden und diese schädigen sowie enzymatische Reaktionen inhibieren. Im Stuhl enthaltene Inhibitorklassen sind - soweit bekannt - Hämoglobin und dessen Metaboliten, Gallensäuren und Gallensäurenderivate sowie Polysaccharide. Im Stand der Technik finden sich verschiedene Lösungsvorschläge für dieses Problem, die jedoch keine befriedigenden Ergebnisse liefern (Deuter et al., Nucleic Acids Res. 1995, 23:3800-3801; van Zwet et al., J. Clin. Microbiol. 1994, 32:1346-1348; Wilde et al., J. Clin Microbiol. 1990, 28:1300-1307; Hopwood et al., Int. J. Legal Med. 1996, 108:237-243; Sidransky et al., Science 1992, 256:103-105; Ramamurthy et al., J. Clin. Micorbiol. 1993, 31: 3068-3070; Monteiro et al., J. Clin. Microbiol. 1997, 35:995-998; Uwatoko et al., Vet. Microbiol. 1996, 52:73-79; Tuchili et al., J. Vet. Med. Sci. 1996, 58:881-884; Pantosti et al., J. Clin. Microbiol. 1997, 35:2482-2486, US 4,935,342; WO 93/20235). In der Regel wird die Probe in alkalischem Milieu aufgearbeitet.

Sivolap et al., Chem. Abstr. 1992, 117:206001 u beschreiben den Zusatz phenolneutralisierender Substanzen bei der Isolierung von DNA aus Pflanzenmaterial. Chung et al., Chem. Abstr. 1997, 127:146699x beschreiben die Extraktion von DNA aus Pflanzenmaterial mit einem basischen Puffer.

US 5817798 beschreibt den Zusatz von DTT zur Aufreinigung von RNA.

In WO 97/07239 wird ein Verfahren zur Reinigung, Stabilisierung oder/und Isolierung von Nukleinsäuren aus biologischen Materialien, insbesondere Fäkalien, beschrieben, bei dem man einer Nukleinsäuren enthaltenden Probe aus biologischen Materialien eine Adsorptionsmatrix zur Bindung von Verunreinigungen zusetzt. Vorzugsweise verwendet man eine Adsorptionsmatrix auf Kohlenhydratbasis, z.B. Stärke, Zellulose, Glycogen, oder/und andere biogene oder nicht biogene Kohlenhydrate oder Mischungen davon, wobei Mehle aus Getreide, Erbsen, Mais, Kartoffeln oder Bestandteile daraus oder Mischungen bevorzugt sind. Mit dem in der WO 97/07239 offenbarten Verfahren werden in manchen Fällen jedoch die Nukleinsäuren schädigenden Substanzen nicht vollständig entfernt.

Eine Weiterentwicklung dieses Verfahrens offenbart die DE 199 00 638. Dort wird ein Verfahren zur Isolierung von Nukleinsäuren aus biologischen Materialien beschrieben, bei dem man der Nukleinsäuren enthaltenden Probe außer der Adsorptionsmatrix zur Bindung von Verunreinigung einen definierten Puffer zusetzt, wobei der Puffer dadurch gekennzeichnet ist, daß er einen sauren bis neutralen pH-Wert sowie einen hohen Salzgehalt hat und eine phenolneutralisierende Substanz enthält. Dieses Verfahren liefert zwar Nukleinsäuren in befriedigender Reinsheit, hat aber den Nachteil, daß die beschriebene Adsorptionsmatrix stark quillt und die Homogenisierung erschwert. Dies ist insbesondere nach Einwirkung von Hitze der Fall, die zur Lyse von Bakterien, Viren und anderen Pathogenen vorteilhaft ist.

Eine Aufgabe der vorliegenden Erfindung war somit die Bereitstellung eines verbesserten Verfahrens zur Aufreinigung von Nukleinsäuren, das die beschriebenen Nachteile der aus dem Stand der Technik bekannten Verfahren überwindet.

Überraschenderweise wurde festgestellt, daß die Aufreinigung von Nukleinsäuren *15*aus inhibitorischen Proben verbessert werden kann, indem ein definierter Puffer verwendet wird, ohne daß der Probe eine wie in der WO 97/07239 oder DE 199 00 638 beschriebene Adsorptionsmatrix zur Bindung von Verunreinigungen zugesetzt werden muß.

Gegenstand der Erfindung ist ein Verfahren zur Reinigung, Stabilisierung oder/und Isolierung von Nukleinsäuren aus Materialproben, wobei man der Nukleinsäuren enthaltenden Probe einen Puffer zusetzt, der
(a) einen pH-Wert von 2 bis 6,5 hat,
(b) eine Salzkonzentration von mindestens 100 mmol/l hat, und
(c) die phenolneutralisierende Substanz Polyvinylpyrrolidon enthält,
wobei das Verfahren dadurch gekennzeichnet ist, daß der Probe, dem Gemisch aus Probe und Puffer oder einem daraus abgeleiteten Homogenat, Lysat oder Extrakt keine Adsorptionsmatrix zur Bindung von Verunreinigungen zugesetzt wird

Solche Adsorptionsmatrices zur Bindung von Verunreinigungen werden in den Patentanmeldungen WO 97/07239 und DE 199 00 638 beschrieben, auf die vollinhaltlich Bezug genommen wird.

Der Puffer hat dabei vorzugsweise eine Salzkonzentration von 100 mmol/l bis maximal zur Löslichkeitsgrenze des verwendeten Salzes. Bevorzugte Bereiche sind 100 mmol/l bis 2,5 mol/l, besonders bevorzugt 250 bis 2,5 mol/l, besonders bevorzugt 300 mmol/l bis 1,5 mol/l, besonders bevorzugt 300 mmol/l bis 700 mmol/l. Ein besonders bevorzugter Bereich ist 400 mmol/l bis 600 mmol/l. Unter Salz sind hier anorganische Salze zu verstehen, insbesondere Alkali- oder Erdalkalisalze von Mineralsäuren. Vorzugsweise wird als Salz ein Alkalihalogenid, z.B. NaCl oder KCl oder Gemische davon verwendet.

In einer weiteren, bevorzugten Ausführungsform wird LiCl als Salz benutzt. Als Konzentration kann 100 mmol/l bis zur Löslichkeitsgrenze eingesetzt werden. Bei der Verwendung von LiCl ist eine Konzentration von 0,5 mol/l bis zur Löslichkeitsgrenze, besonders vorteilhaft von 0,5 bis 2,5 mol/l bevorzugt.

In einer weiteren Ausgestaltungsform enthält der erfindungsgemäße Puffer mindestens 0,1 % (Gew/Vol) eines Detergenz, bevorzugt mindestens 0,5 %. Als Detergens kommt vorzugsweise ein ionisches Detergenz zur Anwendung, insbesondere Natrium-dodecylsulfat (SDS) in einer Konzentration von 0,1 bis 5 % (Gew/Vol), besonders bevorzugt 0,5 bis 5 % (Gew/Vol), ganz besonders bevorzugt 1 bis 2 % (Gew/Vol).

In einer weiteren Ausgestaltungsform kann der erfindungsgemäße Puffer 1 bis 200 mmol/l, vorzugsweise 10 mmol/l oder mehr als 10 mmol/l, besonders bevorzugt mindestens 20 mmol/l eines Chelatbildners enthalten. Ein besonders bevorzugter Bereich ist 25 bis 75 mmol/l. Bevorzugt ist Ethylendiamintetraessigsäure (EDTA) oder ein Salz davon, bevorzugt das Dinatriumsalz.

Der erfindungsgemäße Puffer weist bevorzugt einen pH-Wert von mehr als 3, aber weniger als 6,5, bevorzugt von 4 bis 6,5, besonders bevorzugt von 5 bis 6 auf. Als günstig hat sich dabei die Verwendung von Acetatpuffern, z.B. Essigsäure/Na-Acetat (NaAc) erwiesen. Es können jedoch auch andere Puffer, z. B. Phosphat- oder Citratpuffer, eingesetzt werden.

Der erfindungsgemäße Puffer enthält die phenolneutralisierende Substanz Polyvinylpyrrolidon (Poly(1-vinyl-2-pyrrolidon), PVP, CAS 9003-39-8; zu beziehen z.B. über Sigma-Aldrich Fine Chemicals, St. Louis, MO, USA) in unterschiedlichen Polymerisationsgraden, z.B. PVP-10 (PVP mit einem durchschnittlichen Molekulargewicht von 10.000). Bevorzugt ist ein Puffer, der mindestens 0,5 % Polyvinylpyrrolidon als phenolneutralisierende Substanz enthält. Bevorzugte Konzentrationsbereiche sind 1 - 30 % (Gew/Vol), bevorzugt 2 - 15 %, besonders bevorzugt 4 - 10 % PVP.

Der erfindungsgemäße Puffer ist vorzugsweise ein Lysepuffer, d.h. er hat bevorzugt eine Zusammensetzung, die eine Lyse von Zellen bewirkt, insbesondere eine Lyse der Zellen, die die Nukleinsäuren enthalten, die im gegebenen Fall von Interesse sind. Dem Fachmann sind die Bedingungen bekannt (z.B. Ionen- und/oder Detergenzkonzentrationen), bei denen die Lyse (d.h. Perforation, Zerstörung oder Auflösung der Zellmembranen und/oder Zellwände) der gewünschten Zellen wie Mikroorganismen, tierischen oder pflanzlichen Zellen eintritt.

Die Nukleinsäuren enthaltende Probe stammt bevorzugt aus Materialien, die Nukleinsäure abbauende bzw. enzymatische Reaktionen hemmende Verunreinigungen enthalten. Insbesondere hemmen solche Verunreinigungen die enzymatische Aktivität von Restriktionsenzymen und Enzymen, die für die Polymerase-Kettenreaktion (PCR) verwendet werden. Vorzugsweise stammt die Probe aus Fäkalien. Sie kann jedoch auch aus anderen Quellen stammen, beispielsweise tierischen oder pflanzlichen Geweben, Gewebe- oder Zellkulturen, Knochenmark, humanen und tierischen Körperflüssigkeiten wie Blut, Serum, Plasma, Urin, Sperma, Zerebrospinalflüssigkeit, Sputum und Abstrichen, Pflanzen, Pflanzenteilen und -extrakten, z.B. Säften, Pilzen, prokaryontischen oder eukaryontischen Mikroorganismen wie Bakterien oder Hefen, fossilen oder mumifizierten Proben, Bodenproben, Klärschlamm, Abwässern und Lebensmitteln (insbesondere prozessierten, d.h. technisch aufbereiteten Lebensmitteln). Die Proben können wasserunlösliche Bestandteile enthalten.

Ein weiterer Aspekt der Erfindung betrifft ein Verfahren wie vorstehend beschrieben, bei dem das Gemisch aus Probe und Puffer oder ein daraus abgeleitetes Homogenat, Lysat oder Extrakt bei mindestens 50°C inkubiert wird.

Ein weiterer Aspekt der Erfindung betrifft die Verwendung eines Verfahrens wie vorstehend beschrieben zur Analyse, zum Nachweis oder zur Isolierung von Nukleinsäuren aus Stuhlproben.

Ein weiterer Aspekt der Erfindung betrifft ein Reagenzienkit zur Reinigung, Stabilisierung oder/und Isolierung von Nukleinsäuren aus biologischen Materialien, enthaltend einen Puffer wie vorstehend beschrieben, insbesondere einen Puffer, der
(a) einen pH-Wert von 2 bis 6,5 hat,
(b) eine Salzkonzentration von mindestens 100 mmol/l hat, und
die phenolneutralisierende Substanz Polyvinylpyrrolidon enthält.

Das erfindungsgemäße Reagenzienkit ist dadurch gekennzeichnet, daß es keine Adsorptionsmatrix zur Bindung von Verunreinigungen enthält, wie sie in den Patentanmeldungen WO 97/07239 und DE 199 00 638 beschrieben ist.

Der Puffer kann dabei in fertiger Form, als Konzentrat oder Lyophilisat vorliegen.

Vorzugsweise enthält das Reagenzienkit zusätzliche Mittel zur Reinigung von Nukleinsäuren, die z.B. mineralische oder/und organische Träger sowie gegebenenfalls Lösungen, Hilfsstoffe oder/und Zubehör umfassen. Solche Mittel sind aus dem Stand der Technik bekannt (siehe z.B. WO 95/01359) und kommerziell erhältlich. Mineralische Bestandteile von Trägem können beispielsweise poröse oder nicht-poröse Metalloxide oder Metallmischoxide, z.B. Aluminiumoxid, Titandioxid, Eisenoxid oder Zirkoniumdioxid, Silicagele, Materialien auf Basis von Gläsern, z.B. modifizierte oder nicht-modifizierte Glaspartikel oder Glasmehl, Quarz, Zeolithe oder Mischungen von einer oder mehrerer der oben genannten Substanzen sein. Andererseits kann der Träger auch organische Bestandteile enthalten, die z.B. aus gegebenenfalls mit funktionellen Gruppen modifizierten Latexpartikeln, synthetischen Polymeren, wie etwa Polyethylen, Polypropylen, Polyvinylidenflourid, insbesondere ultrahochmolekularem Polyethylen oder HD-Polyethylen, oder Mischungen von einer oder mehreren der zuvor genannten Substanzen ausgewählt werden.

Der Träger kann beispielsweise in Form von Partikeln mit einer mittleren Größe von 0,1 µm bis 100 µm vorliegen. Bei Verwendung eines porösen Trägers ist eine mittlere Porengröße von 2 µm bis 100 µm bevorzugt. Der Träger kann beispielsweise in Form loser Schüttungen von Partikeln, Filterschichten, z.B. aus Glas, Quarz oder Keramik, Membranen, z.B. Membranen, in denen ein Silicagel angeordnet ist, Fasern oder Geweben aus mineralischen Trägern, wie etwa Quarz oder Glaswolle sowie in Form von Latices oder Frittenmaterialien aus synthetischen Polymeren vorliegen.

Außerdem kann das erfindungsgemäße Reagenzienkit auch Hilfsstoffe wie z.B. eine Protease wie Proteinase K, oder Enzyme und andere Mittel zur Manipulation von Nukleinsäuren enthalten, z.B. mindestens einen Amplifikationsprimer, und zur Amplifikation von Nukleinsäuren geeignete Enzyme, z.B. eine Nukleinsäurepolymerase oder/und mindestens eine Restriktionsendonuklease.

Die Primer zur Amplifikation von Nukleinsäuren stammen zweckmäßigerweise aus den zu analysierenden Genen, d.h. beispielsweise aus Onkogenen, Tumorsuppressorgenen oder/und Mikrosatellitenabschnitten, oder sie sind geeignet zur Amplifikation viraler oder bakterieller Nukleinsäuresequenzen. Zur Amplifikation von Nukleinsäuren geeignete Enzyme und Restriktionsendonukleasen sind bekannt und kommerziell erhältlich.

Ein weiterer Aspekt der Erfindung ist ein Verfahren zur Reinigung, Stabilisierung oder/und Isolierung von Nukleinsäuren aus Materialproben mit den Schritten
(a) Gewinnung einer Probe aus Nukleinsäuren enthaltendem Material,
(b) Zusetzen eines Puffer, der
   einen pH-Wert von 2 bis 6,5 hat,
   eine Salzkonzentration von mindestens 100 mmol/l hat, und
   die phenolneutralisierende Substanz Polyvinylpyrrolidon enthält,
dadurch gekennzeichnet, daß der Probe, dem Gemisch aus Probe und Puffer oder einem daraus abgeleiteten Extrakt, Lysat oder Homogenat keine Adsorptionsmatrix zur Bindung von Verunreinigungen zugesetzt wird.

Bevorzugt handelt es sich dabei um eine Materialprobe, die Nukleinsäure abbauende bzw. enzymatische Reaktionen hemmende Verunreinigungen enthält. Insbesondere hemmen solche Verunreinigungen die enzymatische Aktivität von Nukleinsäure-interagierenden Enzymen, z.B. Nukleasen wie Restriktionsendonukleasen, Reverse Transkriptasen, Nukleinsäure-Polymerasen, Ligasen etc., insbesondere von Enzymen, die für die Polymerase-Kettenreaktion (PCR), LCR (Ligase chain reaction), NASBA (Nucleic Acid Base Specific Amplification) oder 3SR (Self sustained Sequence Replication) verwendet werden. Bevorzugte Ausführungsformen des Puffers sind dabei wie vorstehend beschrieben.

Ein weiterer Aspekt der Erfindung ist ein solches Verfahren mit den Schritten
(a) Gewinnung einer Probe aus Nukleinsäuren enthaltendem Material,
(b) Zusetzen eines Puffers, der einen pH-Wert von 2 bis 6,5, eine Salzkonzentration von mindestens 100 mmol/l hat, und die phenolneutralisierende Substanz Polyvinylpyrrolidon enthält, und
(c) Homogenisierung des Gemisches aus Probe und Puffer.

Ein weiterer Aspekt der Erfindung ist ein solches Verfahren mit den Schritten
(a) Gewinnung einer Probe aus Nukleinsäuren enthaltendem Material,
(b) Homogenisierung des Materials, und
(c) Zusetzen eines Puffers, der einen pH-Wert von 2 bis 6,5, eine Salzkonzentration von mindestens 100 mmol/l hat, und die phenolneutralisierende Substanz Polyvinylpyrrolidon enthält.

Unter Homogenisierung soll eine mechanische, thermische, enzymatische oder chemische Zerkleinerung von Materialproben oder Probenbestandteilen, insbesondere wasserunlöslicher Materialproben, verstanden werden. Zur Homogenisierung können an sich bekannte Verfahren ausgeführt werden, z.B. unter Verwendung eines üblichen Rüttelmischers für Mikrozentrifugationsgefäße (Vortex-Rüttler), eines Ultraturrax oder Polytran, einer French Press, eines Potters, eines Mörsers, oder einer Kugelmühle.

In weiteren Schritten können unlösliche Bestandteile durch Zentrifugation abgetrennt, dem Extrakt eine Protease zugesetzt, oder/und der Extrakt auf ≥ 50°C erhitzt werden.

Die beschriebenen Schritte (Zugabe des erfindungsgemäßen Puffers, Homogenisierung, Zentrifugation, Zugabe einer Protease, Erhitzen), soweit sie ausgeführt werden, können je nach Notwendigkeit in unterschiedlicher Reihenfolge erfolgen. So kann der gewonnenen, ggf. grob zerkleinerten Materialprobe zunächst der erfindungsgemäße Puffer zugesetzt werden, woran sich ein Homogenisierungsschritt und dann ein Zentrifugationsschritt anschließen kann. Hat der Puffer lytische Eigenschaften, müssen dabei andere Zentrifugationsbedingungen angewendet werden, als wenn der Puffer nichtlysierend ist und die Zelllyse erst in einem späteren Verfahrensschritt erreicht wird, da im letzteren Fall darauf geachtet werden muß, daß die Zellen, die die gewünschten Nukleinsäuren enthalten, nicht bei der Zentrifugation verloren gehen. Sollen dagegen beispielsweise Nukleinsäuren aus Viren isoliert werden, die in der Materialprobe extrazellulär vorliegen, kann es wünschenswert sein, einen nichtlytischen Puffer zu verwenden und nach der Zugabe des Puffers und Homogenisierung in der Probe enthaltene Zellen zusammen mit anderen unlöslichen Bestandteilen durch Zentrifugation abzutrennen.

An den Aufschluß der Materialprobe in der oben beschriebenen Weise kann sich eine chromatographische Aufreinigung bzw. Isolierung der Nukleinsäuren in an sich bekannter Weise anschließen, z.B. über eine Silicagelmembran in einer Zentrifugationssäule in Gegenwart chaotroper Salze.

Ein Weg, die Erfindung auszuführen, besteht darin, eine Materialprobe mit dem erfindungsgemäßen Puffer zu versetzen und das Gemisch, sofern notwendig, zu homogenisieren. Zur Homogenisierung kann z.B. ein Vortex-Rüttler verwendet werden. Diese Schritte können bei Raumtemperatur oder, bevorzugt, verringerter Temperatur, z.B. ≤ 10°C, insbesondere ≤ 4°C erfolgen. Nach einer ggf. erfolgten Homogeniserung können unlösliche Bestandteile abgetrennt werden. Sind die Zellen zu diesem Zeitpunkt bereits lysiert, z.B. weil der erfindungsgemäße Puffer lytische Eigenschaften hat, können die unlöslichen Bestandteile z.B. durch Zentrifugation für 1 bis 5 min bei 10.000 bis 25.000 x g, vorzugsweise bei 20.000 x g abgetrennt werden.

Es kann notwendig sein, den so erhaltenen Extrakt bzw. das so erhaltene Lysat unter Bedingungen zu inkubieren, die für eine Freisetzung der Nukleinsäuren aus dem Probenmaterial förderlich sind. Solche Inkubationsbedingungen werden insbesondere dann verwendet, wenn Nukleinsäuren aus "schwer" aufschließbaren Materialien, z.B. Bakterien oder Parasiten oder Viren nachgewiesen werden sollen. In diesem Fall kann durch chemische, thermische und/oder enzymatische Behandlung die Freisetzung der Nukleinsäuren verbessert werden, wodurch eine höhere Ausbeute an Nukleinsäuren aus dem Probenmaterial sowohl hinsichtlich Gesamt-DNA als auch spezifisch hinsichtlich der nachzuweisenden DNA erhalten wird. Vorzugsweise wird hierbei eine Temperaturerhöhung, z.B. auf ≥ 50°C, insbesondere auf ≥ 70°C vorgenommen.

Wenn andererseits Nukleinsäuren aus leicht aufschließbaren Materialien, z.B. empfindlichen Zellen wie etwa Humanzellen bestimmt werden sollen, kann es vorteilhaft sein, den Prozeß der Extraktion und des Zellaufschlusses bei verringerter Temperatur, z.B. ≤ 10°C, insbesondere ≤4°C auszuführen, um auf diese Weise die unerwünschte Freisetzung anderer Nukleinsäuren in der Probe zu vermeiden oder einzuschränken.

Die Behandlung mit dem erfindungsgemäßen Puffer führt zu einer sehr guten Stabilität der in der Probe enthaltenen Nukleinsäuren und bei einer anschließenden Isolierung der Nukleinsäuren zu einer besseren Reproduzierbarkeit, ohne die zusätzlichen Schritte der Zugabe, Inkubation und anschließender Abtrennung einer Adsorptionsmatrix zur Bindung von Verunreinigungen vornehmen zu müssen. Dies gilt insbesondere, wenn sich an die Isolierung eine enzymatische Manipulation der Nukleinsäuren mit Nukleinsäure-interagierenden Enzymen anschließt, z.B. Nukleasen wie Restriktionsendonukleasen, Reverse Transkriptasen, Nukleinsäure-Polymerasen, Ligasen etc. Besonders bevorzugt ist das erfindungsgemäße Verfahren, wenn anschließend eine Amplifikation und/oder Restriktionsspaftung, z.B. Amplifikation durch PCR, LCR (Ligase chain reaction), NASBA (Nucleic Acid Base Specific Amplification) oder 3SR (Self sustained Sequence Replication) durchgeführt wird.

Ein besonders bevorzugter Aspekt der vorliegenden Erfindung ist die Analyse, der Nachweis oder die Isolierung von Nukleinsäuren, insbesondere DNA, aus Stuhlproben. Durch das erfindungsgemäße Verfahren sind auf vereinfachte Weise saubere und amplifizierbare Nukleinsäuren aus fäkalen Proben erhältlich, die zum diagnostischen Nachweis von Infektionen, insbesondere bakteriellen oder Virusinfektionen, oder von Mutationen, insbesondere von tumorspezifischen DNA-Mutationen verwendet werden können.

Die vorliegende Erfindung soll durch die nachfolgenden Abbildungen und Beispiele erläutert werden.

### Abbildungen

**Abbildung 1:** Ausbeuten der DNA-Isolierung gemäß Verfahren V2 und V3 aus Beispiel 2 aus verschiedenen Stuhlproben. Die Werte stellen Mittelwerte und mittlere Abweichung von den Gesamtausbeuten in µg dar. Die ersten beiden Nummern der Abszissenbeschriftung kodieren die jeweilige Stuhlprobe. Die Bezeichnung m.T. (mit Tablette) bezeichnet das Verfahren V2 aus Beispiel 2, o.T. (ohne Tablette) das Verfahren V3.
**Abbildung 2:** Inhibition der PCR-Reaktion durch DNA-Präparationen gemäß Verfahren V2 und V3 aus Beispiel 2 aus verschiedenen Stuhlproben. Die ersten beiden Nummern der Abszissenbeschriftung kodieren die jeweilige Stuhlprobe. Die Bezeichnung m.T. bezeichnet das Verfahren V2 aus Beispiel 2, o.T. das Verfahren V3. Zur Berechnung des Wertes delta Ct siehe Beispiel 2. Die PCR-Reaktion wurde in Anwesenheit von 10 ng BSA/µl ausgeführt (BSA = Rinderserumalbumin).
**Abbildung 3:** Ausbeuten der DNA-Isolierung gemäß Verfahren V2 und V3 aus Beispiel 2 aus verschiedenen Stuhlproben. Die Werte stellen Mittelwerte von den Gesamtausbeuten in µg dar. Die ersten beiden Nummern der Abszissenbeschriftung kodieren die jeweilige Stuhlprobe. Die Bezeichnung m.T. bezeichnet das Verfahren V2 aus Beispiel 2, o.T. das Verfahren V3.
**Abbildung 4:** Inhibition der PCR-Reaktion durch DNA-Präparationen gemäß Verfahren V1, V2 und V3 aus Beispiel 2 aus verschiedenen Stuhlproben. Die ersten beiden Nummern der Abszissenbeschriftung kodieren die jeweilige Stuhlprobe. Die Bezeichnung m.T. bezeichnet das Verfahren V2 aus Beispiel 2, o.T. das Verfahren V3. Zur Berechnung des Wertes delta Ct siehe Beispiel 2. Die PCR-Reaktion wurde in Anwesenheit von 10 ng BSA/µl ausgeführt (BSA = Rinderserumalbumin).
**Abbildung 5:** Ausbeuten der DNA-Isolierung gemäß Verfahren V1, V2 und V3 aus Beispiel 2 aus verschiedenen Stuhlproben. Die Werte stellen Mittelwerte von den Gesamtausbeuten in µg dar. Die ersten beiden Nummern der Abszissenbeschriftung kodieren die jeweilige Stuhlprobe. Die Bezeichnung m.T. bezeichnet das Verfahren V2 aus Beispiel 2, o.T. das Verfahren V3, und m.P. das Verfahren V1.
**Abbildung 6:** Inhibition der PCR-Reaktion durch DNA-Präparationen gemäß Verfahren V1, V2 und V3 aus Beispiel 2 aus verschiedenen Stuhlproben. Die ersten beiden Nummern der Abszissenbeschriftung kodieren die jeweilige Stuhlprobe. Die Bezeichnung m.T. bezeichnet das Verfahren V2 aus Beispiel 2, o.a. das Verfahren V3, und m.P. das Verfahren V1. Zur Berechnung des Wertes delta Ct siehe Beispiel 2. Die PCR-Reaktion wurde in Anwesenheit von 100 ng BSA/µl ausgeführt (BSA = Rinderserumalbumin).
**Abbildung 7:** Ausbeuten der DNA-Isolierung gemäß dem Verfahren aus Beispiel 3 in Abhängigkeit von der PVP-Konzentration des Puffers P1. Wert in µg Gesamtausbeute. PVP-Konzentration in % (Gew/Vol). Stuhlprobe 27. Doppelbestimmung.
**Abbildung 8:** Inhibition der PCR-Reaktion durch DNA-Präparationen gemäß dem Verfahren aus Beispiel 3 in Abhängigkeit von der PVP-Konzentration des Puffers P1. Stuhlprobe 27. 1 ng/µl BSA.
**Abbildung 9:** Ausbeuten der DNA-Isolierung gemäß dem Verfahren aus Beispiel 3 in Abhängigkeit von der PVP-Konzentration des Puffers P1. Wert in µg Gesamtausbeute. PVP-Konzentration in % (Gew/Vol). Stuhlprobe 61. Doppelbestimmung.
**Abbildung 10:** Inhibition der PCR-Reaktion durch DNA-Präparationen gemäß dem Verfahren aus Beispiel 3 in Abhängigkeit von der PVP-Konzentration des Puffers P1. Stuhlprobe 61. 1 ng/µl BSA.
**Abbildung 11:** Ausbeuten der DNA-Isolierung gemäß dem Verfahren aus Beispiel 3 in Abhängigkeit vom pH-Wert des Puffers P1. Wert in µg Gesamtausbeute. Stuhlprobe 51. Doppelbestimmung.
**Abbildung 12:** Inhibition der PCR-Reaktion durch DNA-Präparationen gemäß dem Verfahren aus Beispiel 3 in Abhängigkeit vom pH-Wert des Puffers P1. Stuhlprobe 51. 1 ng/µl BSA.

### Beispiele

### Beispiel 1: DNA-Isolierung aus Stuhlproben

Menschliche Stuhlproben wurden gesammelt, eingefroren und bei -20°C aufbewahrt. 200 mg Stuhl wurden in einem 2 ml-Mikrozentrifugengefäß abgewogen und auf Eis gekühlt. Dann wurde die Stuhlprobe in 1600 µl Puffer P1 (100 mmol/l NaAc pH 5,5, 50 mmol/l EDTA, 500 mmol/l NaCl, 2 % (Gew/Vol) PVP-10, 1,4 % (Gew/Vol) SDS) aufgenommen und die Mischung durch Vortexbehandlung für 1 min homogenisiert. Das Lysat wurde für 3 min zur Präzipitation von Stuhlpartikeln und anderen Verunreinigungen bei 20.000 x g (14.000 rpm in der verwendeten Zentrifuge Eppendorf 5417C; Rotor FA 45-30-11) zentrifugiert. 1250 µl des Überstandes wurden in ein neues 2 ml-Mikrozentrifugengefäß überführt und durch Invertieren gemischt. 600 µl dieses Lysats wurden in ein neues 2 ml-Mikrozentrifugationsgefäß überführt und mit einem kommerziell erhältlichen DNA-Reinigungskit (QIAamp^{®} DNA Mini Kit, Qiagen GmbH, Hilden, Deutschland) nach den Herstellerangaben weiter aufgereinigt. Diese Reinigungsschritte schlossen die Zugabe von Proteinase-K, eine Inkubation von 10 min bei 70°C sowie die chromatographische Reinigung der Nukleinsäuren über eine Silicagelmembran in einer Zentrifugationssäule in Gegenwart chaotroper Salze ein. Das dabei erhaltene DNA-Eluat hatte ein Volumen von 200 µl.

### Beispiel 2: DNA-Isolierung aus Stuhlproben mit und ohne Verwendung einer Kohlenhydratmatrix

Menschliche Stuhlproben wurden gesammelt, eingefroren und bei -20°C aufbewahrt. DNA wurde aus insgesamt 16 verschiedenen Stuhlproben nach verschiedenen Verfahren isoliert sowie anschließend mit Hilfe einer PCR-Methode ("Realtime quantitative PCR"; SFV-TaqMan^{®}-Reaktion; SFV = Semliki Forest Virus; Wang et Brown, Anal. Biochem. 1999, 269:198-201; de Kok et al., Clin. Chem. 1998, 44:2201-2204) quantitativ auf Inhibitoren untersucht.

V1-V3: 1600 mg Stuhl wurden in ein 50 ml Zentrifugenröhrchen gegeben und auf Eis gekühlt. Dann wurde die Stuhlprobe in 12,8 ml Puffer P1 (100 mmol/l NaAc pH 5,5, 50 mmol/l EDTA, 500 mmol/l NaCl, 2 % (Gew/Vol) PVP-10, 1,4 % (Gew/Vol) SDS) aufgenommen und die Mischung durch Vortexbehandlung für 1 min homogenisiert. Zur Pelletierung nicht löslicher Bestandteile im Stuhl erfolgte eine Zentrifugation für 3 min bei 5000 rpm (entspricht 5338 g in der verwendeten Zentrifuge Sigma 4K15, Rotor 1156). Der Überstand wurde in ein neues 50 ml Zentrifugenröhrchen überführt und erneut für 3 min bei 5000 rpm zentrifugiert. Der Überstand wird nachfolgend in ein weiteres 50 ml Zentrifugenröhrchen überführt und durch Invertieren gemischt. Das so erhaltene Lysat wird in Aliquots von 1400 µl in 2 ml-Mikrozentrifugengefäßen aufgeteilt für die folgenden drei Verfahren verwendet:
V1: 500 mg einer aus Kartoffelmehl und Zellulose bestehenden, **pulverförmigen** Adsorptionsmatrix (im Mischungsverhältnis 3:1 [Gew/Gew]) wurden dem Lysat zugefügt und durch Vortexbehandlung für 1 min resuspendiert.
V2: 500 mg einer aus Kartoffelmehl und Zellulose bestehenden, **tablettenförmigen** Adsorptionsmatrix (im Mischungsverhältnis 3:1 [Gew/Gew]; plus Mg-Stearat als Tablettierungshilfstoff) wurden dem Lysat zugefügt und durch Vortexbehandlung für 1 min resuspendiert.
V3: Dem Lysat wurde keine Adsorptionsmatrix zugefügt.

Die weitere Prozessierung in allen drei Verfahren war identisch. Die Suspension (V1 und V2) bzw. das unbehandelte Lysat (V3) wurden für 3 min zur Präzipitation von Stuhlpartikeln, der Adsorptionsmatrix und anderen Verunreinigungen bei 20.000 x g (14.000 rpm in der verwendeten Zentrifuge, siehe Beispiel 1) zentrifugiert. Der Überstand wurde in ein neues Mikrozentrifugationsgefäß überführt und für weitere 3 min zentrifugiert. 600 µl des Überstands der zweiten Zentrifugation wurden in ein neues 2 ml-Mikrozentrifugationsgefäß überführt und mit einem kommerziell erhältlichen DNA-Reinigungskit (QIAamp^{®} DNA Mini Kit, Qiagen GmbH, Hilden, Deutschland) nach den Herstellerangaben weiter aufgereinigt. Diese Reinigungsschritte schlossen die Zugabe von Proteinase-K, eine Inkubation von 10 min bei 70°C sowie die chromatographische Reinigung der Nukleinsäuren über eine Silicagelmembran in einer Zentrifugationssäule in Gegenwart chaotroper Salze ein. Das dabei erhaltene DNA-Eluat hatte ein Volumen von 200 µl.

Die DNA-Eluate aus den Verfahren V1, V2, und V3 wurden mit Hilfe einer SFV-TaqMan-Reaktion (TaqMan^{®}, Perkin Elmer Biosystems, Foster City, CA, USA) quantitativ auf Inhibitoren untersucht (PCR über 50 Cyclen). Dazu wurden einem PCR-Mastermix pro Ansatz 1000 Kopien des Plasmids pSFV1 (Gibco BRL Life Technologies, Inc., Gaithersburg, MD, USA) zugefügt, welches stuhlfremde Sequenzen des Semliki Forest Virus trägt. Das Amplikon ist im nsP1-Leseraster des Plasmids lokalisiert. Der Mastermix enthält: desweiteren 1x TaqMan Buffer A (Perkin Eimer), 3 mmoV1 MgCl2 (Perkin Elmer), 200 µM dATP, dCTP, dGTP, 400 µM dUTP (Perkin Elmer), je 300 µM Primer, 100 µM Probe, 1-10 ng/µl BSA (New England Biolabs BSA007), 0,025 U/µl Amplitaq Gold (Perkin Elmer) und 0,01 U/µl UNG (Perkin Elmer). Im Gesamtvolumen von 25 µl waren außerdem 5 µl DNA-Eluat aus Stuhl enthalten. Waren inhibitorische Substanzen im Stuhl enthalten, wurde die Amplifikation des SFV-Amplikons verzögert (steigende Ct-Werte) und im Extremfall vollständig inhibiert (Ct = 50). Als Positivkontrolle wurde anstelle der DNA-Eluate 5 µl des in allen Verfahren verwendeten Elutionspuffers P2 (10 mmol/l Tris/HCl pH 9.0, 0,5 mmol/l EDTA) verwendet (Ct-Werte von ca. 30). Jede DNA-Isolierung erfolgte in Doppelwerten. Von jedem Eluat wurden 3 TaqMan-Reaktionen angesetzt. Aus den so erhaltenen 6 Ct-Werten wurden Mittelwerte berechnet (MW-Ct). Das Maß der Inhibition wurde als Differenz aus MW-Ct_{Eluat} minus MW-Ct_{P2} (= ΔCt, delta Ct) berechnet. Nicht inhibitorische Eluate weisen demnach ΔCt-Werte von 0 (in der Praxis ± 1), vollständig inhibitorische DNA-Eluate hingegen ΔCt-Werte von 16 bis 20 auf.

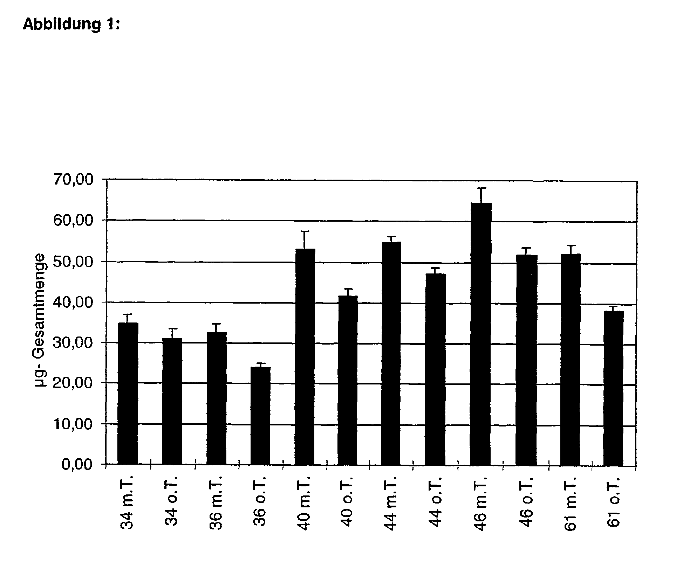

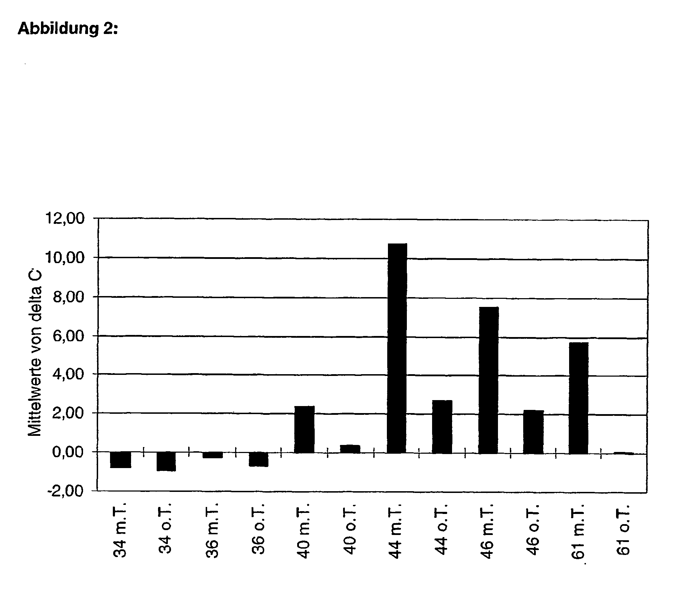

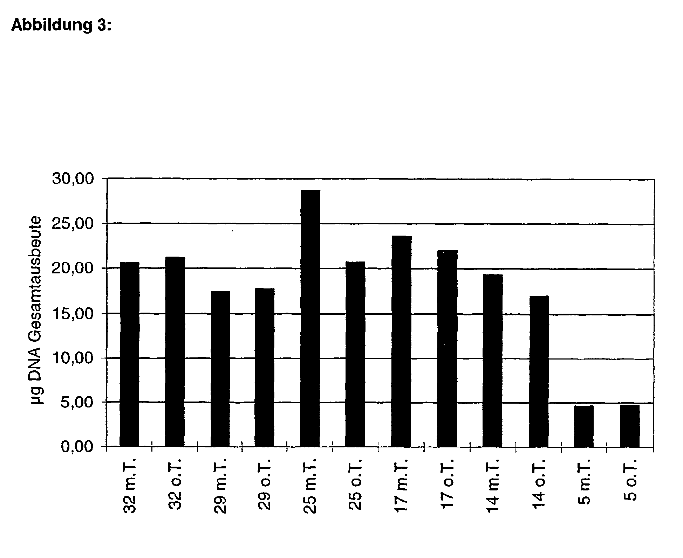

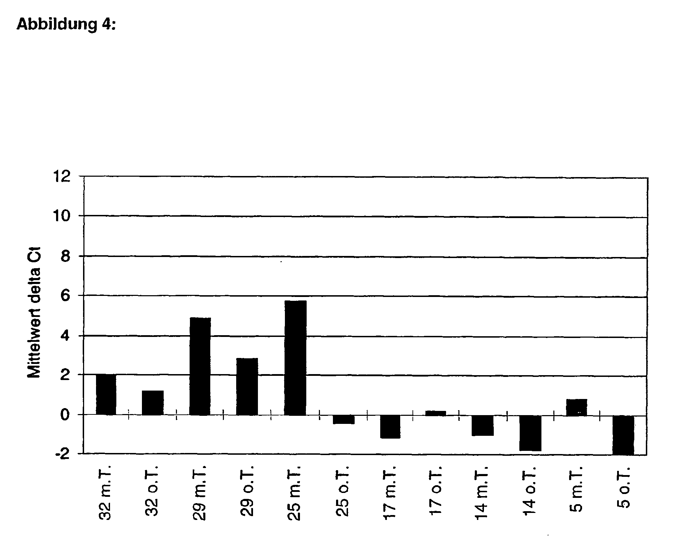

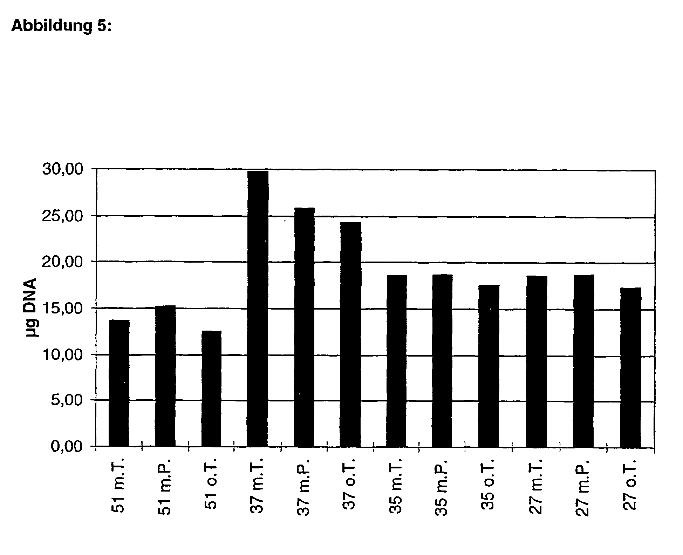

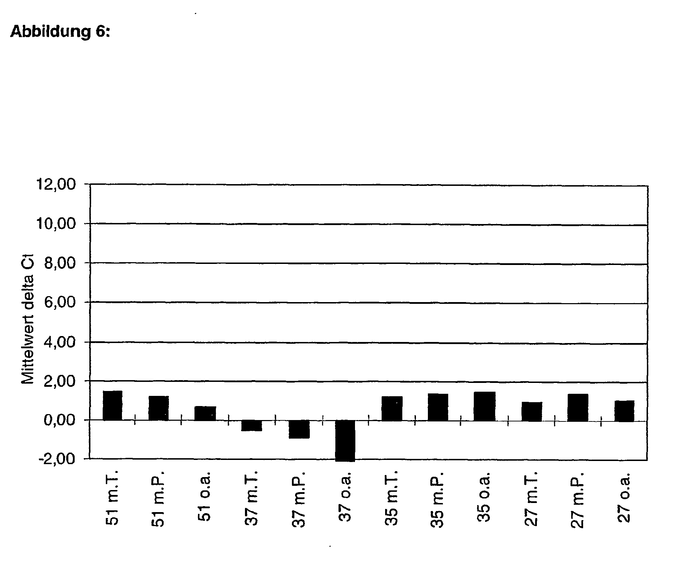

Überraschenderweise zeigte sich, daß es für die Abtrennung inihibitorischer Verunreinigungen entbehrlich ist, bei der Probenaufarbeitung eine Adsorptionsmatrix zuzufügen (Verfahren V1, V2), wenn man den erfindungsgemäßen Puffer benutzt (Verfahren V3). Die Ergebnisse für Gesamtausbeute und Inhibitorgehalt zeigen die Abbildungen 1 bis 6.

### Beispiel 3: DNA-Isolierung aus Stuhlproben in Abhängigkeit verschiedener Parameter des Puffers

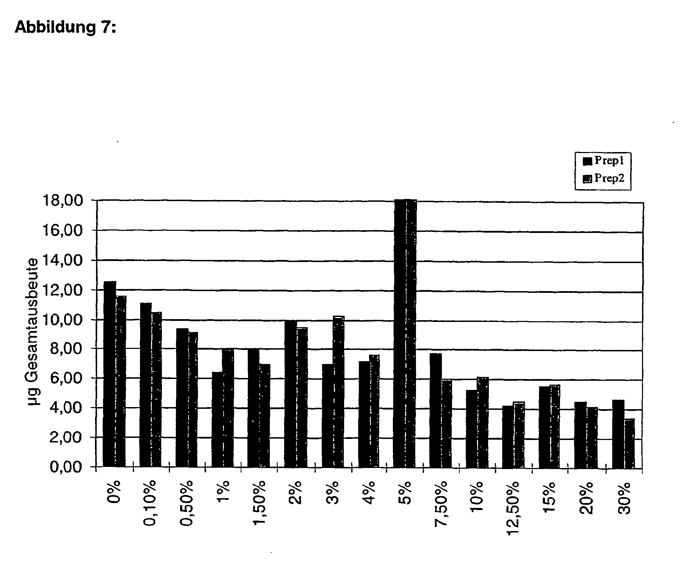

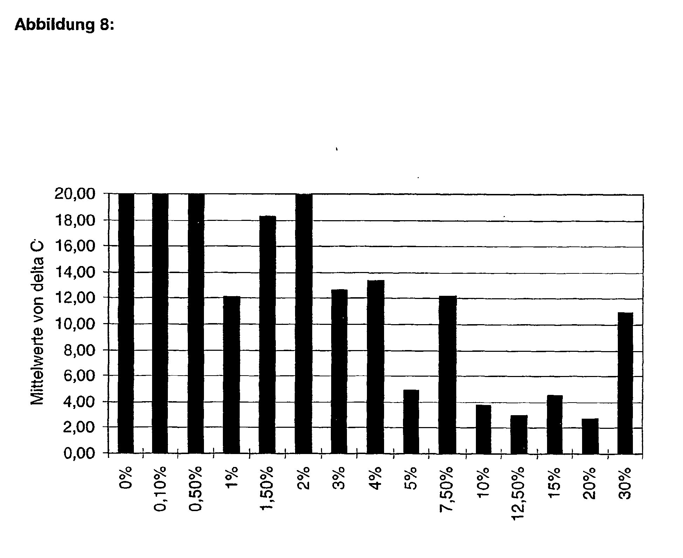

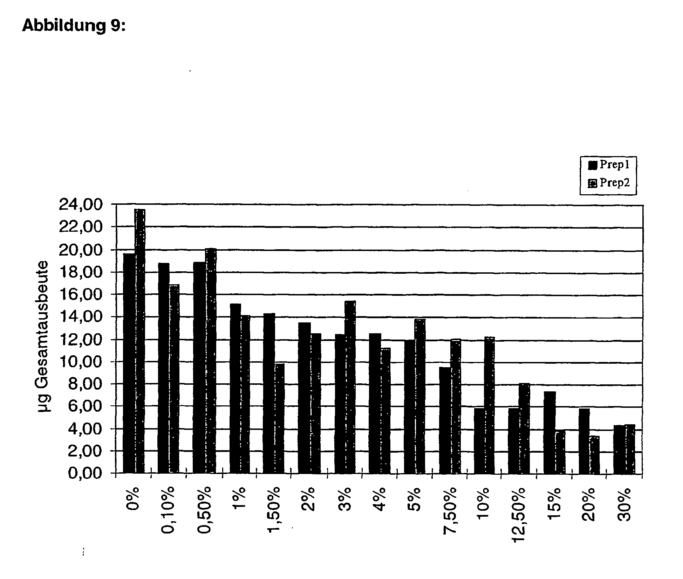

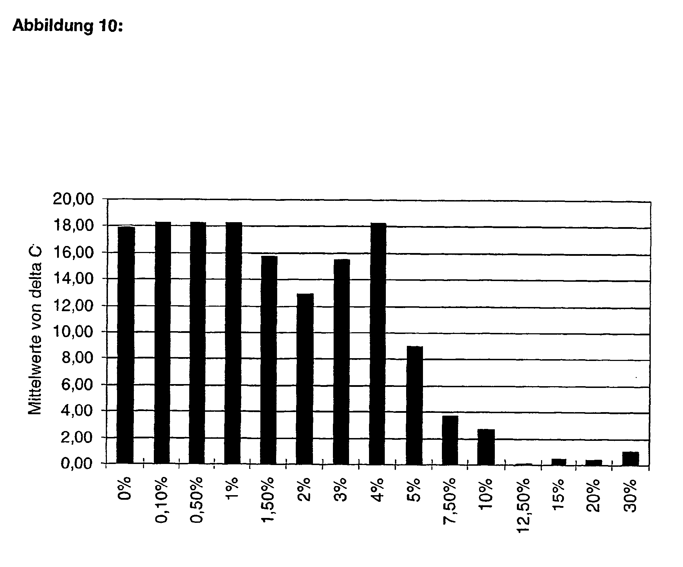

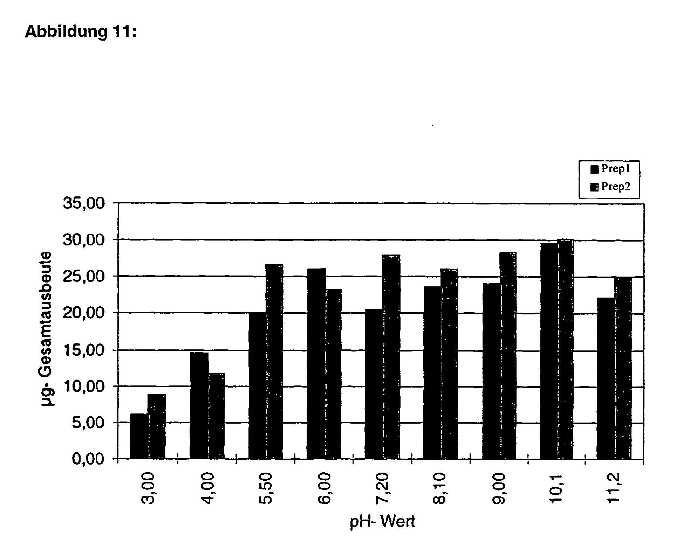

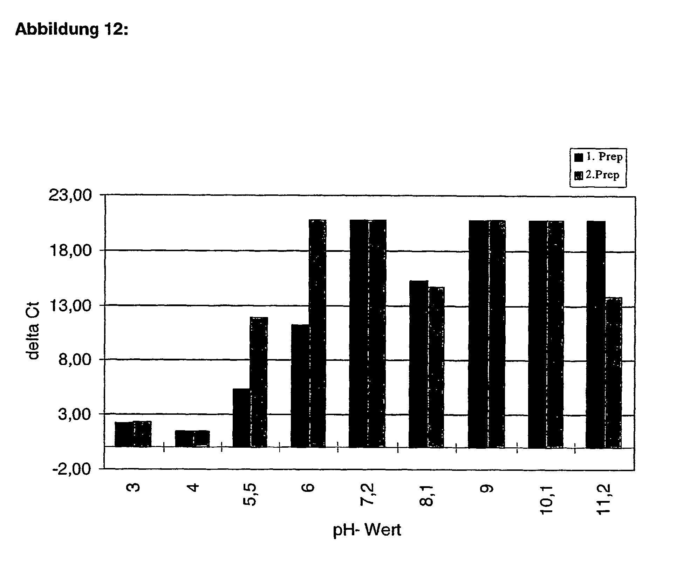

DNA wurde aus Stuhlproben gemäß dem Verfahren aus Beispiel 1 isoliert. Dabei wurde im Puffer P1 in einer Serie von Experimenten die Konzentration an Polyvinylpyrrolidon variiert (0 bis 30 %), in einer zweiten Serie von Experimenten der pH-Wert des Puffers (pH 3 bis 11,2). Die Ergebnisse für Gesamtausbeute und Inhibitorgehalt zeigen die Abbildungen 7 bis 12.

## Patentansprüche

1. Verfahren zur Reinigung, Stabilisierung oder/und Isolierung von Nukleinsäuren aus Materialproben, wobei man der Nukleinsäuren enthaltenden Probe einen Puffer zusetzt, der
(a) einen pH-Wert von 2 bis 6,5 hat,
(b) eine Salzkonzentration von mindestens 100 mmol/l hat, und
(c) die phenolneutralisierende Substanz Polyvinylpyrrolidon enthält,
**dadurch gekennzeichnet, dass** der Probe, dem Gemisch aus Probe und Puffer oder einem daraus abgeleiteten Homogenat, Lysat oder Extrakt keine Adsorptionsmatrix zur Bindung von Verunreinigungen zugesetzt wird.

2. Verfahren nach Anspruch 1, **dadurch gekennzeichnet, dass** es einen weiteren Schritt (d) Homogenisierung des Gemisches aus Probe und Puffer oder Homogenisierung der Probe vor der Zugabe des Puffers umfasst.

3. Verfahren nach Anspruch 1 oder 2, **dadurch gekennzeichnet, dass** der Puffer eine Salzkonzentration von mindestens 250 mmol/l, vorzugsweise mindestens 300 mmol/l hat.

4. Verfahren nach einem der Ansprüche 1 bis 3, **dadurch gekennzeichnet, dass** das im Puffer enthaltene Salz LiCl, NaCl oder/und KCI ist.

5. Verfahren nach Anspruch 4, **dadurch gekennzeichnet, dass** das im Puffer enthaltene Salz LiCl ist und in einer Konzentration von 0,5 bis zur Löslichkeitsgrenze, bevorzugt 0,5 bis 2,5 mol/l vorliegt.

6. Verfahren nach einem der Ansprüche 1 bis 5, **dadurch gekennzeichnet, dass** der Puffer zusätzlich mindestens 0,1 % (Gew/Vol) eines Detergenz enthält.

7. Verfahren nach einem der Ansprüche 1 bis 6, **dadurch gekennzeichnet, dass** der Puffer zusätzlich mindestens 10 mmol/l, vorzugsweise mindestens 20 mmol/l eines Chelatbildners enthält.

8. Verfahren nach einem der Ansprüche 1 bis 7, **dadurch gekennzeichnet, dass** der Puffer einen pH-Wert von mehr als 3, aber weniger als 7, bevorzugt von 4 bis 6,5, besonders bevorzugt von 5 bis 6 hat.

9. Verfahren nach einem der Ansprüche 1 bis 8, **dadurch gekennzeichnet, dass** der Puffer mindestens 0,5 % (Gew/Vol) Polyvinylpyrrolidon als Substanz (c) enthält.

10. Verfahren nach Anspruch 9, **dadurch gekennzeichnet, dass** die Konzentration von Polyvinylpyrrolidon im Puffer 1-30 % (Gew/Vol), bevorzugt 2-15 %, besonders bevorzugt 4-10 % beträgt.

11. Verfahren nach einem der Ansprüche 1 bis 10, **dadurch gekennzeichnet, dass** die Nukleinsäuren enthaltende Probe aus Fäkalien, tierischen oder pflanzlichen Geweben, Gewebe- oder Zellkulturen, Knochenmark, humanen und tierischen Körperflüssigkeiten wie Blut, Serum, Plasma, Urin, Sperma, Zerebrospinalflüssigkeit, Sputum und Abstrichen, Pflanzen, Pflanzenteilen und -extrakten, z.B. Säften, Pilzen, prokaryontischen oder eukaryontischen Mikroorganismen wie Bakterien oder Hefen, fossilen oder mumifizierten Proben, Bodenproben, Klärschlamm, Abwässern oder Lebensmitteln stammt.

12. Verfahren nach einem der Ansprüche 1 bis 11, bei dem das Gemisch aus Probe und Puffer oder ein daraus abgeleitetes Homogenat oder Extrakt bei mindestens 50 °C inkubiert wird.

13. Verwendung eines Verfahrens nach einem der Ansprüche 1 bis 12 zur Analyse, zum Nachweis oder zur Isolierung von Nukleinsäuren aus Stuhlproben.

14. Reagenzienkit zur Reinigung, Stabilisierung oder/und Isolierung von Nukleinsäuren aus biologischen Materialien, enthaltend einen zur Aufnahme einer Nukleinsäure enthaltenden Probe geeigneten Puffer wie in einem der Ansprüche 1 bis 8 definiert, nicht aber eine Adsorptionsmatrix zur Bindung von Verunreinigungen.

## Claims

1. Process for purifying, stabilising and/or isolating nucleic acids from material samples, wherein a buffer is added to the sample containing the nucleic acids, said buffer
(a) having a pH of 2 to 6.5,
(b) having a salt concentration of at least 100 mmol/l, and
(c) containing the phenol-neutralising substance polyvinylpyrrolidone,
**characterised in that** no adsorption matrix for binding impurities is added to the sample, the mixture of sample and buffer or any homogenisate, lysate or extract derived therefrom.

2. Process according to claim 1, **characterised in that** it comprises a further step (d) of homogenising the mixture of sample and buffer or homogenising the sample before the addition of the buffer.

3. Process according to claim 1 or 2, **characterised in that** the buffer has a salt concentration of at least 250 mmol/l, preferably at least 300 mmol/l.

4. Process according to one of claims 1 to 3, **characterised in that** the salt contained in the buffer is LiCl, NaCl and/or KCl.

5. Process according to claim 4, **characterised in that** the salt contained in the buffer is LiCl and is present in a concentration of 0.5 up to the limit of solubility, preferably 0.5 to 2.5 mol/l.

6. Process according to one of claims 1 to 5, **characterised in that** the buffer additionally contains at least 0.1% (wt/vol) of a detergent.

7. Process according to one of claims 1 to 6, **characterised in that** the buffer additionally contains at least 10 mmol/l, preferably at least 20 mmol/l of a chelating agent.

8. Process according to one of claims 1 to 7, **characterised in that** the buffer has a pH of more than 3 but less than 7, preferably from 4 to 6.5, more preferably from 5 to 6.

9. Process according to one of claims 1 to 8, **characterised in that** the buffer contains at least 0.5% (wt/vol) of polyvinylpyrrolidone as substance (c).

10. Process according to claim 9, **characterised in that** the concentration of polyvinylpyrrolidone in the buffer is 1-30% (wt/vol), preferably 2-15%, more preferably 4-10%.

11. Process according to one of claims 1 to 10, **characterised in that** the sample containing nucleic acids is derived from faecal material, animal or plant tissue, tissue or cell cultures, bone marrow, human and animal body fluids such as blood, serum, plasma, urine, sperm, cerebrospinal fluid, sputum and smears, plants, plant parts and extracts, e.g. saps, fungi, prokaryotic or eukaryotic microorganisms such as bacteria or yeasts, fossilised or mummified samples, soil samples, clarified sludge, sewage or foodstuffs.

12. Process according to one of claims 1 to 11, wherein the mixture of sample and buffer or a homogenisate or extract derived therefrom is incubated at at least 50°C.

13. Use of a process according to one of claims 1 to 12 for the analysis, detection or isolation of nucleic acids from stool samples.

14. Reagent kit for purifying, stabilising and/or isolating nucleic acids from biological materials, containing a buffer suitable for absorbing a sample containing nucleic acid as defined in one of claims 1 to 8, but not an adsorption matrix for binding impurities.

## Revendications

1. Procédé de purification, de stabilisation et/ou d'isolement d'acides nucléiques à partir d'échantillons, dans lequel on ajoute à l'échantillon contenant des acides nucléiques un tampon qui
(a) possède une valeur de pH allant de 2 à 6,5,
(b) a une concentration en sel d'au moins 100 mmoles/l, et
(c) contient la substance polyvinylpyrrolidone qui neutralise le phénol,
**caractérisé en ce qu'**aucune matrice d'adsorption pour la liaison des impuretés n'est ajoutée à l'échantillon, au mélange constitué de l'échantillon et du tampon ou d'un homogénat, lysat ou extrait qui en est dérivé.

2. Procédé selon la revendication 1, **caractérisé en ce qu'**il comprend une deuxième étape (d) d'homogénéisation du mélange constitué de l'échantillon et du tampon ou d'homogénéisation de l'échantillon avant l'ajout du tampon.

3. Procédé selon la revendication 1 ou 2, **caractérisé en ce que** le tampon présente une concentration en sel d'au moins 250 mmoles/l, de préférence au moins 300 mmoles/l.

4. Procédé selon l'une quelconque des revendications 1 à 3, **caractérisé en ce que** le sel contenu dans le tampon est du LiCl, NaCl et/ou KCl.

5. Procédé selon la revendication 4, **caractérisé en ce que** le sel contenu dans le tampon est du LiCl et est présente dans une concentration de 0,5 jusqu'à la limite de solubilité, de préférence de 0,5 à 2,5 moles/l.

6. Procédé selon l'une quelconque des revendications 1 à 5, **caractérisé en ce que** le tampon contient en plus au moins 0,1 % (poids/volume) d'un détergent.

7. Procédé selon l'une quelconque des revendications 1 à 6, **caractérisé en ce que** le tampon contient au moins en plus 10 mmoles/l, de préférence au moins 20 mmoles/l d'un agent chélateur.

8. Procédé selon l'une quelconque des revendications 1 à 7, **caractérisé en ce que** le tampon a une valeur de pH supérieure à 3, mais inférieure à 7, de préférence de 4 à 6,5, de façon particulièrement préférée de 5 à 6.

9. Procédé selon l'une quelconque des revendications 1 à 8, **caractérisé en ce que** le tampon contient au moins 0,5 % (poids/volume) de polyvinylpyrrolidone comme substance (c).

10. Procédé selon la revendication 9, **caractérisé en ce que** la concentration de polyvinylpyrrolidone dans le tampon est de 1-30 % (poids/volume), de préférence de 2-15 %, de façon particulièrement préférée de 4-10 %.

11. Procédé selon l'une quelconque des revendications 1 à 10, **caractérisé en ce que** l'échantillon contenant les acides nucléiques provient de matières fécales, de tissus animaux ou végétaux, de cultures tissulaires ou cellulaires, de moelle osseuse, de fluides corporels humains ou animaux tels que le sang, le sérum, le plasma, l'urine, le sperme, le liquide céphalorachidien, le crachat ou le frottis, des plantes, des parties ou extraits de plantes, par exemple, de la sève, des champignons, des microorganismes procaryotes ou eucaryotes tels que des bactéries ou des levures, des échantillons fossiles ou momifiés, des échantillons de sol, des boues d'épuration, des eaux usées ou des aliments.

12. Procédé selon l'une quelconque des revendications 1 à 11, dans lequel le mélange d'échantillon et de tampon ou bien un homogénat ou extrait qui en est dérivé est incubé au moins à 50°C.

13. Utilisation d'un procédé selon l'une quelconque des revendications 1 à 12 pour l'analyse, la détection ou l'isolement d'acides nucléiques à partir d'échantillons de selles.

14. Kit réactif pour la purification, la stabilisation et/ou l'isolement d'acides nucléiques à partir de matières biologiques, contenant un tampon approprié destiné à recueillir un échantillon contenant un acide nucléique tel que défini dans l'une quelconque des revendications 1 à 8, mais pas de matrice d'adsorption pour la liaison d'impuretés.
